# EUROPEAN PATENT SPECIFICATION

(11) **EP 2 073 642 B1**
(45) Date of publication and mention of the grant of the patent: **11.11.2015**
(21) Application number: 07820635.6
(22) Date of filing: 27.09.2007
(51) Int. Cl.: A23K 1/165, C12N 9/24

(54) **XYLANASES FOR ANIMAL FEED**
XYLANASEN FÜR TIERFUTTER
XYLANASES POUR ALIMENTS POUR ANIMAUX

(30) Priority: 29.09.2006 DK 200601262
(43) Date of publication of application: 01.07.2009
(73) Proprietor: Novozymes A/S, 2880 Bagsværd (DK); DSM IP Assets B.V., 6411 TE Heerlen (NL)
(72) Inventor: FISCHER, Morten, DK-1720 Copenhagen V (DK); PETTERSSON, Dan, DK-3540 Lynge (DK)
(86) International application number: PCT/EP2007/060242
(87) International publication number: WO 2008/037757

(56) References cited:
- WO-A-01/66711
- WO-A-03/106654
- WO-A-2005/079585
- US-A- 5 736 384

## Description

### Background of the Invention

### Field of the Invention

This invention relates to the field of animal feed.

Cereal grains are important components of animal feed. They contain, i.a., plant polysaccharides which can function as a major nutritional component in the diet (e.g. starch), but also a number of non-starch polysaccharides (NSP) including, among others, arabinoxylans. Major farm animals like poultry and pigs lack the relevant enzymes in their digestive tracts for digesting the NSP. It is known in the art to use xylanases in animal feed in order to improve the feed utilization.

The present invention relates to the use in animal feed of a xylanase having a percentage of identity to a xylanase from Paenibacillus (amino acids 1-182 of SEQ ID NO: 2) of at least 90%. The invention is defined by the claims.

### Description of the Related Art

WO 2006/083240 discloses, in Example 6, the use in chicken feed of a xylanase designated XylA1A. The amino acid sequence of XylA1A corresponds to SEQ ID NO: 2 in WO 2006/083240, which is identical to the mature part of UNIPROT:Q6TLP3 which is included in the present sequence listing as SEQ ID NO: 9. According to the UNIPROT database entry this sequence derives from a bacterium isolated from an environmental sample. The percentage identity of the xylanase of SEQ ID NO: 9 to amino acids 1-184 of SEQ ID NO: 4 is below 82.7%.

The RONOZYME WX xylanase is a known mono-component animal feed xylanase derived from Thermomyces lanuginosus and commercially available from DSM Nutritional Products, Wurmisweg 576, CH-4303 Kaiseraugst, Switzerland. This xylanase and its use in animal feed are also described in WO 96/23062. This xylanase does not have a molecular weight below 24 kDa.

A xylanase from Paenibacillus pabuli having the amino acid sequence of amino acids 1-182 of SEQ ID NO: 2 herein, and its use in a process for preparing a dough-based product, are described in WO 2005/079585.

The amino acid sequence of a xylanase from Paenibacillus sp. KCTC 8848P was submitted to the public Uniprot database with accession no. UNIPROT:Q9F9B9, and is included in the present sequence listing as SEQ ID NO: 4.

WO 97/13853 discloses (SEQ ID NO: 6) a xylanase from Aspergillus niger which is identical except for one amino acid to the sequence of amino acids 1-188 of SEQ ID NO: 6 herein ("xyl II" from Aspergillus niger).

WO 2004/018662 discloses (as SEQ ID NO: 9 in WO 2004/018662) another xylanase from Aspergillus niger which is identical to SEQ ID NO: 8 herein ("xyl III" from Aspergillus niger).

Chesson et al, in J. Sci. Food Agric. 1997, 75, 289-295, report studies of cell wall porosity and available surface area of wheat straw and wheat grain fractions.

It is an object of the present invention to improve the solubilization and/or degradation of insoluble non-starch polysaccharides (NSP) such as arabinoxylans with a view to improving the nutritional value of animal feed, e.g. by improving the feed conversion ratio (FCR), the growth rate, and/or the weight gain.

### Summary of the Invention

The present invention relates to the use in animal feed of a xylanase having a percentage of identity to amino acids
1-182 of SEQ ID NO: 2 of at least 90%, the percentage of identity being determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage.

The invention also relates to the use of such xylanase in the preparation of a composition for use in animal feed.

The invention furthermore relates to an animal feed composition comprising such xylanase and (a) at least one fat soluble vitamin; (b) at least one water soluble vitamin; and/or (c) at least one trace mineral.

Still further, the invention relates to such an animal feed composition having a crude protein content of 50 to 800 g/kg and comprising such xylanase, as well as a method for improving the nutritional value of an animal feed, wherein such xylanase is added to the feed.

Finally, the invention relates to the use of such xylanase for the solubilization and/or degradation of non-starch polysaccharides during gastric and intestinal digestion; and for pre-treatment of animal feed or animal feed components.

### Detailed Description of the Invention

In what follows, the expression "xylanase of the invention" refers to a xylanase for use according to the invention, as described herein.

### EC Classes of Enzymes - Bernard Henrissat Glycoside Hydrolase Families

Enzymes can be classified on the basis of the handbook Enzyme Nomenclature from NC-IUBMB, 1992), see also the ENZYME site at the internet: http://www.expasy.ch/enzyme/. ENZYME is a repository of information relative to the nomenclature of enzymes. It is primarily based on the recommendations of the Nomenclature Committee of the International Union of Biochemistry and Molecular Biology (IUB-MB) and it describes each type of characterized enzyme for which an EC (Enzyme Commission) number has been provided (Bairoch A. The ENZYME database, 2000, Nucleic Acids Res 28:304-305). This IUB-MB Enzyme nomenclature is based on their substrate specificity and occasionally on their molecular mechanism; such a classification does not reflect the structural features of these enzymes.

Another classification of certain glycoside hydrolase enzymes, such as endoglucanase, xylanase, galactanase, mannanase, dextranase and alpha-galactosidase, in families based on amino acid sequence similarities has been proposed a few years ago. They currently fall into 90 different families: See the CAZy(ModO) internet site (Coutinho, P.M. & Henrissat, B. (1999) Carbohydrate-Active Enzymes server at: http://afmb.cnrs-mrs.fr/-cazy/CAZY/index.html (corresponding papers: Coutinho, P.M. & Henrissat, B. (1999) Carbohydrate-active enzymes: an integrated database approach. In "Recent Advances in Carbohydrate Bioengineering", H.J. Gilbert, G. Davies, B. Henrissat and B. Svensson eds., The Royal Society of Chemistry, Cambridge, pp. 3-12; Coutinho, P.M. & Henrissat, B. (1999) The modular structure of cellulases and other carbohydrate-active enzymes: an integrated database approach. In "Genetics, Biochemistry and Ecology of Cellulose Degradation"., K. Ohmiya, K. Hayashi, K. Sakka, Y. Kobayashi, S. Karita and T. Kimura eds., Uni Publishers Co., Tokyo, pp. 15-23).

### Xylanase

For the present purposes, a xylanase means a protein, or a polypeptide, having xylanase activity.

Xylanase activity can be measured using any assay, in which a substrate is employed, that includes 1,4-beta-D-xylosidic endo-linkages in xylans. Assay-pH and assay-temperature are to be adapted to the xylanase in question. Examples of assay-pH-values are pH 4, 5, 6, 7, 8, 9, 10, or 11. Examples of assay-temperatures are 30, 35, 37, 40, 45, 50, 55, 60, 65, 70 or 80°C.

Different types of substrates are available for the determination of xylanase activity e.g. Xylazyme cross-linked arabinoxylan tablets (from MegaZyme), or insoluble powder dispersions and solutions of azo-dyed arabinoxylan.

For assaying xylanase in feed, premix and the like samples, the enzyme is extracted at temperatures ranging from 50°C up to 70°C (with the higher temperatures used for the more thermostable enzymes) in an extraction medium typically consisting of a phosphate buffer (0.1 M and a pH adjusted to the pH optima of the enzyme in question) for a time period of 30 to 60 min. A preferred xylanase assay is disclosed in Example 4.

All measurements are based on spectrophotometric determination principles at approx. 590-600 nm. The enzyme, or the extracted enzyme, as applicable, is incubated with a known amount of substrate and the colour release is measured relative to a standard curve obtained by adding known amounts of an enzyme standard to a similar control diet without enzyme. When no control feed is available, a known amount of enzyme is added to the sample (spiking) and from the differences in response between spiked and non-spiked sample the added amount of enzyme can be calculated.

In a particular embodiment, the xylanase is an enzyme classified as EC 3.2.1.8 (see the ENZYME site referred to above). The official name is endo-1,4-beta-xylanase. The systematic name is 1,4-beta-D-xylan xylanohydrolase. Other names may be used, such as endo-(1-4)-beta-xylanase; (1-4)-beta-xylan 4-xylanohydrolase; endo-1,4-xylanase; xylanase; beta-1,4-xylanase; endo-1,4-xylanase; endo-beta-1,4-xylanase; endo-1,4-beta-D-xylanase; 1,4-beta-xylan xylanohydrolase; beta-xylanase; beta-1,4-xylan xylanohydrolase; endo-1,4-beta-xylanase; beta-D-xylanase. The reaction catalyzed is the endohydrolysis of 1,4-beta-D-xylosidic linkages in xylans.

According to the CAZy(ModO) site referred to above, xylanases are presently classified in either of the following Glycoside Hydrolyase Families: 5, 8, 10, 11, 16, 43, or 62. E.g., GH Family 11 glycoside hydrolases can be characterized as follows:

| | |
|---|---|
| CAZy Family: | Glycoside Hydrolase Family 11 |
| Known Activities: | Xylanase (EC 3.2.1.8) |
| Mechanism: | Retaining |
| Catalytic Nucleophile/Base: | Glu (experimental) |
| Catalytic Proton Donor: | Glu (experimental) |
| 3D Structure Status: | Available (see PDB). |
| Fold: | Beta-jelly roll |
| Clan: | GH-C |

In particular, the xylanase is i) a xylanase of Glycoside Hydrolyase (GH) Family 5, 8, 10, 11, 16, 43, or 62, preferably of GH Family 10, or 11, more preferably of GH Family 11. The expression "of Glycoside Hydrolase Family NN" means that the xylanase in question is or can be classified in GH family "NN" (e.g. 10, or 11).

In another particular embodiment, the xylanase of the invention is derived from a bacterial xylanase, preferably from a bacterium of (i) the phylum of Firmicutes; (ii) the class of Bacilli; (iii) the order of Bacillales; (iv) the family of Paenibacillaceae; or (v) the genus of Paenibacillus; even more preferably from a bacterium of (vi) the species of Paenibacillus pabuli, Paenibacillus polymyxa, or Paenibacillus sp.; most preferably from (vii) strains of Paenibacillus pabuli, or Paenibacillus polymyxa.

The expression "xylanase derived from a bacterial (or Firmicutes, ..., or Paenibacillus pabuli) xylanase" as used hereinabove includes any wild-type xylanase isolated from the bacterium in question, as well as variants or fragments thereof which retain xylanase activity.

The term "variant" refers to a xylanase which comprises a substitution, deletion, and/or insertion of one or more amino acids as compared to the specified xylanase. The variant may be a natural variant (allelic variant), or prepared synthetically. Preferably, amino acid changes are of a minor nature, e.g., conservative amino acid substitutions that do not significantly affect the folding and/or activity of the protein; small deletions; small amino- or carboxyl-terminal extensions, such as an amino-terminal methionine residue; a small linker peptide; or a small extension that facilitates purification by changing net charge or another function, such as a poly-histidine tract, an antigenic epitope or a binding domain.

A "fragment" of a specified xylanase has one or more amino acids deleted from the amino and/or carboxyl terminus of the amino acid sequence of the xylanase.

For purposes of the above definitions of variant and fragment, the term "small" as well as the term "one or more" refer to a maximum of 30 changes as compared to the specified xylanase. In preferred embodiments of either of these definitions, the number of changes is below 30, 25, 20, 15, 10, or below 5.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

In addition to the 20 standard amino acids, non-standard amino acids (such as 4-hydroxyproline, 6-N-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity (i.e., xylanase activity) to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309:59-64. The identities of essential amino acids can also be inferred from analysis of identities with polypeptides which are related to a polypeptide according to the invention.

Single or multiple amino acid substitutions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochem. 30:10832-10837; U.S. Patent No. 5,223,409; WO 92/06204), and region-directed mutagenesis (Derbyshire et al., 1986, Gene 46:145; Ner et al., 1988, DNA 7:127).

Mutagenesis/shuffling methods can be combined with high-throughput, automated screening methods to detect activity of cloned, mutagenized polypeptides expressed by host cells. Mutagenized DNA molecules that encode active polypeptides can be recovered from the host cells and rapidly sequenced using standard methods in the art. These methods allow the rapid determination of the importance of individual amino acid residues in a polypeptide of interest, and can be applied to polypeptides of unknown structure.

In a further particular embodiment the xylanase of the invention is derived from a fungal xylanase. The above definition of "derived from" (in the context of bacterial xylanases) is applicable by analogy also to fungal xylanases. Fungal xylanases include yeast and filamentous fungal xylanases. In preferred embodiments, the xylanase is derived from a fungus of (i) the phylum of Ascomycota; (ii) the class of Pezizomycotina; (iii) the order of Eurotiomycetes; (iv) the sub-order of Eurotiales; (v) the family of Trichocomaceae, preferably the mitosporic Trichocomaceae; even more preferably from a fungus of (vi) the genus Aspergillus; most preferably from (vii) strains of Aspergillus niger. It will be understood that the definition of the aforementioned species includes both the perfect and imperfect states, and other taxonomic equivalents e.g., anamorphs, regardless of the species name by which they are known. Those skilled in the art will readily recognize the identity of appropriate equivalents.

Strains of the abovementioned bacteria and fungi are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

Questions relating to taxonomy can be solved by consulting a taxonomy data base, such as the NCBI Taxonomy Browser which is available at the following internet site: http://www.ncbi.nlm.nih.gov/Taxonomy/taxonomyhome.html/. However, preferably reference is had to the following handbooks: Dictionary of the Fungi, 9th edition, edited by Kirk, P.M., P.F. Cannon, J.C. David & J.A. Stalpers, CAB Publishing, 2001; and Bergey's Manual of Systematic Bacteriology, Second edition (2005).

The term "a" as used herein in whatever context means "one or more", preferably "at least one". This is the case, e.g., for the use in claim 1 of "a" xylanase as specified, which is considered equivalent to claiming the use of "at least one" or "one or more" such xylanases.

The term a "mature" polypeptide or mature amino acid sequence refers to that part of an amino acid sequence which remains after a potential signal peptide part and a potential propeptide part have been cleaved off. Some variation may be observed in the mature parts of enzymes, depending on i.a. expression hosts and fermentation conditions. E.g., experience shows that sometimes also minor C-terminal truncations occur during the secretion process. The term mature part as used herein also takes into account such C-terminal truncations, if any. While the mature peptide part may be identified by computer programs known in the art (e.g. SignaIP 3.0, see J. D. Bendtsen et al, J. Mol. Biol., 340:783-795, 2004), preferably it is identified by determination of the N-terminal, and preferably also C-terminal, of the expressed and secreted, if relevant, xylanase enzyme. E.g., according to our observations, the mature part of the xylanase of SEQ ID NO: 2 is amino acids 1-182 thereof, and the mature part of the xylanase of SEQ ID NO: 4 is amino acids 1-184 thereof.

In a particular embodiment the xylanase of the invention is isolated, i.e. essentially free of other polypeptides of enzyme activity, e.g., at least about 20% pure, preferably at least about 40% pure, more preferably about 60% pure, even more preferably about 80% pure, most preferably about 90% pure, and even most preferably about 95% pure, as determined by SDS-PAGE. As it is generally known in the art, for detection purposes the SDS-gel can be stained with Coomassie or silver staining. It should be ensured that overloading has not occurred, e.g. by checking linearity by applying various concentrations in different lanes on the gel. Such polypeptide preparations are in particular obtainable using recombinant methods of production, whereas they are not so easily obtained and also subject to a much higher batch-to-batch variation when the polypeptide is produced by traditional fermentation methods.

The polypeptides comprised in the composition of the invention are preferably also purified. The term purified refers to a protein-enriched preparation, in which a substantial amount of low molecular components, typical residual nutrients and minerals originating from the fermentation, have been removed. Such purification can e.g. be by conventional chromatographic methods such as ion-exchange chromatography, hydrophobic interaction chromatography and size exclusion chromatography (see e.g. Protein Purification, Principles, High Resolution Methods, and Applications. Editors: Jan-Christer Janson, Lars Rydén, VCH Publishers, 1989). Example 2 of WO 2005/079585 describes a suitable procedure for the purification of the Paenibacillus pabuli xylanase, expressed from Bacillus subtilis.

The use of an isolated and/or purified polypeptide according to the invention is advantageous. For instance, it is much easier to correctly dose enzymes that are essentially free from interfering or contaminating other enzymes. The terms correctly dose refer in particular to the objective of obtaining consistent and constant animal feeding results, and the capability of optimizing dosage based upon the desired effect.

### Identity

The relatedness between two amino acid sequences is described by the parameter "identity".

In particular embodiments, the degree of identity is at least 90%, 95%, 97%, or at least 99%. In still further embodiments, the degree of identity is at least 91%, 92%, 93%, 94%, 96%, or at least 98%.

The invention in particular relates to the use in animal feed of a xylanase having a percentage of identity to amino acids 1-182 of SEQ ID NO: 2 of at least 90%.

In still further particular embodiments, the xylanase of the invention comprises (preferably has, or consists of) a mature part of any one of the xylanase of SEQ ID NO: 2, or a variant or fragment thereof that has xylanase activity.

For purposes of the present invention, the degree of identity between two amino acid sequences is determined on the basis of an alignment of the two amino acid sequences made by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of the present invention ("invention sequence"; e.g. amino acids 1-182 of SEQ ID NO: 2 and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap (in the alignment example below this is represented by "|"). The length of a sequence is the number of amino acid residues in the sequence (e.g. the length of amino acids 1-182 of SEQ ID NO: 2 is 182).

In the purely hypothetical alignment example below, the overlap is the amino acid sequence "HTWGER-NL" of Sequence 1; or the amino acid sequence "HGWGEDANL" of Sequence 2. In the example a gap is indicated by a "-".

Hypothetical alignment example:

Sequence 2: HGWGEDANLAMNPS 14

In this hypothetical example, the number of exact matches is 6. The length of the shortest sequence is 12. Accordingly the degree of identity of Sequence 1 to Sequence 2 is 50%.

In a particular embodiment, the percentage of identity of an amino acid sequence of a polypeptide with, or to, amino acids 1 to 182 of SEQ ID NO: 2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage.

### Animal Feed

The present invention is also directed to methods for using the xylanase of the invention in animal feed, as well as to feed compositions and feed additives comprising it.

The term animal includes all animals, including human beings. Examples of animals are non-ruminants, and ruminants. Ruminant animals include, for example, animals such as sheep, goat, and cattle, e.g. cow such as beef cattle and dairy cows. In a particular embodiment, the animal is a non-ruminant animal. Non-ruminant animals include mono-gastric animals, e.g. pig or swine (including, but not limited to, piglets, growing pigs, and sows); poultry such as turkeys, ducks and chickens (including but not limited to broiler chicks, layers); fish (including but not limited to salmon, trout, tilapia, catfish and carp); and crustaceans (including but not limited to shrimp and prawn).

In particular embodiments, the xylanase of the invention is for use in feed for (i) non-ruminant animals; preferably (ii) mono-gastric animals; more preferably (iii) pigs, poultry, fish, and crustaceans; or, most preferably, (iv) pigs and poultry.

The xylanase of the invention can be fed to the animal before, after, or simultaneously with the diet. The latter is preferred.

The term feed, feed composition, or diet means any compound, preparation, mixture, or composition suitable for, or intended for intake by an animal. More information about animal feed compositions is found below.

Cereal grains are important components of animal feed. Cereal grains contain plant polysaccharides, some of which, e.g. starch, can function as a major nutritional component of the diet. But cereal grains also contain various kinds of non-starch polysaccharides (NSP), which cannot be utilized by non-ruminant animals such as poultry and pigs.

Examples of NSP are xylans, arabinoxylans, beta-glucans, and cellulose. The type and amount of NSP vary from cereal to cereal. The following are examples of approximate NSP content (%, w/w, dry matter) of various cereal grains: Pearled rice 1%, sorghum 5%, maize 8%, wheat 11%, rye 13%, triticale 16%, and barley 17%. For wheat, triticale and maize, arabinoxylans make up more than 50% of the NSP, whereas for barley, sorghum, rye, and rice the arabinoxylans make up approximately 25-45% of the NSP, i.e. still a substantial amount.

For arabinoxylans and beta-glucans, a distinction is made between soluble and insoluble polysaccharides. The terms soluble and insoluble are known in the art and refer to water solubility/insolubility, in particular to the form (soluble/insoluble) of these polysaccharides a) under digestive conditions, b) under intestinal conditions (in the small intestine), or preferably c) after an in vitro procedure as outlined in Example 1 (i.e., 1.5 hours at pH 3.0 and 40°C in the presence of pepsin, and 4.5 hours at pH 6.8 and 40°C in the presence of pancreatin).

Insoluble arabinoxylans are associated with the encapsulation of nutrients such as starch and protein. This encapsulation allows valuable nutrients to by-pass the digestion. When insoluble arabinoxylans are also digested, or solubilized, an improved exposure of nutrients results.

In a particular embodiment, the xylanase of the invention is capable of solubilizing insoluble fibre polysaccharides, such as NSP. Accordingly, the invention relates to the use of a xylanase of the invention for the solubilization of (otherwise insoluble) non-starch polysaccharides during gastric and intestinal digestion. Preferred non-starch polysaccharides are arabinoxylans (arabinoxylan polysaccharides).

The term polysaccharide is known in the art to designate saccharides with 10 or more monosaccharides (see e.g. Food Chemistry, 3rd edition, Springer Verlag, ISBN 3-540-40817-7, Belitz, Grosch, Schieberle (editors), section 4.3.1 on p. 294), in other words, with a degree of polymerization (DP) of at least 10.

Polysaccharides with a DP of at least 10 can be distinguished from oligosaccharides with a DP below 10 as is known in the art, e.g. by Gel filtration on Biogel P-2 in supernatants obtained after 80% ethanol precipitation (see "The Uppsala method for rapid analysis of total dietary fiber" by Theander et al, in particular Fig. 2 on p. 277, in New Developments in Dietary Fiber, Furda and Brine (editors), Plenum Press, 1990, p. 273-281).

In particular, the xylanase of the invention it is capable of reducing the amount of insoluble xylans and arabinoxylans in an in vitro model mimicking the gastric and small intestinal digestion steps in monogastric digestion - as described in Examples 1, 2, and 5 herein. Preferably, the amount of residual (i.e., after incubation with xylanase) insoluble arabinoxylans is not higher than 85% (w/w), more preferably not higher than 84, 83, 82, 81, 80, 79, 78, 77, 76, 75, 74, 73, 72, 71, or 70% (w/w) relative to a control without added xylanase enzyme (100%). This corresponds to a reduction of the amount of insoluble arabinoxylans of at least 15%, preferably at least 16, 17, 18, 19, 20, 21, 22, 23, 24, 25, 26, 27, 28, 29, or at least 30% (w/w) relative to a control without added xylanase enzyme (0%).

In still further particular embodiments, the conditions of the in vitro model are: (i) substrate (diet): 0.35 g wheat, 0.21 g barley, 0.13 g soy bean meal, and 0.11 g wheat bran, provided as a premixed diet, milled to pass a 0.5 mm screen; (ii) a gastric step incubation in which the diet is incubated with 0.1 ml of the xylanase to be tested together with 4.1 ml 0.072 M HCl for 1.5 hours and with 0.5 ml 0.072 M HCl/pepsin (Sigma P-7000; 3000 U/g diet) for 1 hour (i.e. 30 min HCl-substrate premixing) at pH 3.0 and 40°C; (iii) a subsequent intestinal step incubation with 0.9 ml 0.215M NaOH plus 0.4 ml 1M NaHCO₃ and pancreatin 8 mg/g diet for 4 hours (Sigma P-7545) at pH 6.8-7.0 and 40°C; followed by (iv) a determination of the amount of residual insoluble arabinoxylan, e.g. using the Uppsala method, as described in Example 1 and 5.

The invention also relates to the use of a xylanase of the invention for the degradation of non-starch polysaccharides during gastric and intestinal digestion. Preferred non-starch polysaccharides are fiber polysaccharides, in particular arabinoxylans (arabinoxylan polysaccharides).

In particular, the xylanase of the invention it capable of degrading xylan and arabinoxylan polysaccharides in an in vitro model mimicking the gastric and small intestinal digestion steps in monogastric digestion - as described in Example 5 herein. Preferably, the amount of residual (i.e., after incubation with xylanase) total arabinoxylans is not higher than 94% (w/w), more preferably not higher than 93, 92, 91, 90, 89, 88, 87, 86, 85, 84, 83, 82, 81, or 80% (w/w) relative to a control without added xylanase enzyme (100%).

In still further particular embodiments, the conditions of the in vitro model are: (i) substrate (diet): 0.35 g wheat, 0.21 g barley, 0.13 g soy bean meal, and 0.11 g wheat bran, provided as a premixed diet, milled to pass a 0.5 mm screen; (ii) a gastric step incubation in which the diet is incubated with 0.1 ml of the xylanase to be tested together with 4.1 ml 0.072 M HCl for 1.5 hours and with 0.5 ml 0.072 M HCl/pepsin (Sigma P-7000; 3000 U/g diet) for 1 hour (i.e. 30 min HCl-substrate premixing) at pH 3.0 and 40°C; (iii) a subsequent intestinal step incubation with 0.9 ml 0.215M NaOH plus 0.4 ml 1M NaHCO₃ and pancreatin 8 mg/g diet for 4 hours (Sigma P-7545) at pH 6.8-7.0 and 40°C; followed by (iv) a determination of the amount of residual total arabinoxylan, e.g. using the Uppsala method, as described in Example 5.

The dosage of the xylanase of the invention can be optimized using simple trial-and-error methods as is known in the art. Different xylanases may have different optimum dosage ranges. Examples of suitable dosage ranges are: 0.1-500 mg enzyme protein (EP)/kg diet (substrate); preferably 0.2-400, 0.3-300, 0.4-200, or 0.5-100 mg EP/kg diet. Other preferred dosage ranges are 0.6-90, 0.7-80, 0.7-70, 1-70, 2-70, 3-70, 4-70, 5-70, 6-70, or 7-70 - all in mg EP/kg diet. Still further preferred enzyme dosages are from 10-500, 10-400, 10-300, 10-200, 10-100, 10-90, 10-80, or 10-70 - all in mg EP/kg diet. The amount of xylanase enzyme protein (EP) may be determined as described in Example 1.

For determining mg xylanase enzyme protein per kg feed, the xylanase is purified from the feed composition, and the specific activity of the purified xylanase is determined using a relevant assay. The xylanase activity of the feed composition as such is also determined using the same assay, and on the basis of these two determinations, the dosage in mg xylanase enzyme protein per kg feed is calculated.

The same principles apply for determining mg xylanase enzyme protein in feed additives. Of course, if a sample is available of the xylanase used for preparing the feed additive or the feed, the specific activity is determined from this sample (no need to purify the xylanase from the feed composition or the additive).

The term improving the nutritional value of an animal feed means improving the availability of nutrients, whereby the growth rate, weight gain, and/or feed conversion (i.e. the weight of ingested feed relative to weight gain) of the animal is/are improved.

The xylanase can be added to the feed in any form, be it as a purified and/or isolated xylanase, or in admixture with other components intended for addition to animal feed, i.e. in the form of animal feed additives, such as the so-called pre-mixes for animal feed.

In a further aspect the present invention relates to compositions for use in animal feed, such as animal feed, and animal feed additives, e.g. premixes.

Apart from the xylanase of the invention, the animal feed additives of the invention contain at least one fat-soluble vitamin, and/or at least one water soluble vitamin, and/or at least one trace mineral. Macro-minerals are also usually included in feed additives.

Further, optional, feed-additive ingredients are colouring agents, e.g. carotenoids such as beta-carotene, astaxanthin, and lutein; aroma compounds; stabilisers; antimicrobial peptides; polyunsaturated fatty acids; reactive oxygen generating species; and/or at least one other enzyme selected from amongst another xylanase (EC 3.2.1.8); and/or beta-glucanase (EC 3.2.1.4 or EC 3.2.1.6).

Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.

Examples of antifungal polypeptides (AFP's) are the Aspergillus giganteus, and Aspergillus niger peptides, as well as variants and fragments thereof which retain antifungal activity, as disclosed in WO 94/01459 and WO 02/090384.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and enzymes such as an oxidase, an oxygenase or a syntethase.

Usally fat- and water-soluble vitamins, as well as trace minerals form part of a so-called premix intended for addition to the feed, whereas macro minerals are usually separately added to the feed. A premix enriched with a xylanase of the invention is an example of an animal feed additive of the invention.

In a particular, the animal feed additive is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.

The following are non-exclusive lists of examples of these components:
Examples of fat-soluble vitamins are vitamin A, vitamin D3, vitamin E, and vitamin K, e.g. vitamin K3.
Examples of water-soluble vitamins are vitamin B12, biotin and choline, vitamin B1, vitamin B2, vitamin B6, niacin, folic acid and panthothenate, e.g. Ca-D-panthothenate.
Examples of trace minerals are manganese, zinc, iron, copper, iodine, selenium, and cobalt.
Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.

In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

WO 01/58275 corresponds to US Patent No. 6,960,462.

An animal feed composition according to the invention has a crude protein content of 50-800 g/kg (preferably 50-600 g/kg, more preferably 60-500 g/kg, even more preferably 70-500, and most preferably 80-400 g/kg) and furthermore comprises at least one xylanase as claimed herein. In additional preferred embodiments, the crude protein content is 150-800, 160-800, 170-800, 180-800, 190-800, or 200-800 - all in g/kg (dry matter).

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg) = N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-25% fish meal; and/or 0-25% meat and bone meal; and/or 0-20% whey.

Animal diets can e.g. be manufactured as mash feed (non-pelleted) or pelleted feed. Typically, the milled feed-stuffs are mixed and sufficient amounts of essential vitamins and minerals are added according to the specifications for the species in question. Enzymes can be added as solid or liquid enzyme formulations. For example, a solid enzyme formulation is typically added before or during the mixing step; and a liquid enzyme preparation is typically added after the pelleting step. The enzyme may also be incorporated in a feed additive or premix, as described above.

### Additional particular embodiments

These are additional particular embodiments of the invention:
The use in animal feed of a xylanase having a molecular weight by SDS-PAGE below 24 kDa, wherein the xylanase has a degree of identity to amino acids 1-182 of SEQ ID NO: 2 of at least 90%. The xylanase may also have a percentage of identity to any one of amino acids 1-182 of SEQ ID NO: 2,. In particular embodiments, the degree of identity is at least 90%, 95%, 97%, or at least 99%. The xylanase may be a bacterial xylanase, preferably obtainable from a bacterial strain of the genus Paenibacillus, or a variant, or fragment thereof.

The invention furthermore relates to the use of such xylanase in the preparation of a composition for use in animal feed, as well as compositions comprising such xylanase and (a) at least one fat soluble vitamin, (b) at least one water soluble vitamin, and/or (c) at least one trace mineral.

The invention also relates to an animal feed composition having a crude protein content of 50 to 800 g/kg and comprising such xylanase, as well as a method for improving the nutritional value of an animal feed, wherein such xylanase is added to the feed.

The invention also relates to the use of a xylanase as defined above in the preparation of a composition for use in animal feed.

The invention also relates to a composition comprising a xylanase as defined above, and (a) at least one fat soluble vitamin; (b) at least one water soluble vitamin; and/or (c) at least one trace mineral. The composition preferably further comprises at least one enzyme selected from the following group of enzymes: another xylanase, and/or beta-glucanase. The composition is preferably an animal feed additive.

The invention also relates to an animal feed composition having a crude protein content of 50 to 800 g/kg and comprising a xylanase as defined above.

The invention also relates to a method for improving the nutritional value of an animal feed, wherein a xylanase as defined above, or a composition as defined above, is added to the feed.

The invention also relates to the use of a xylanase as defined above for pre-treatment of animal feed or animal feed components.

### Molecular weight

The xylanase may have a MW below 24 kDa. In particular embodiments, the MW is below 23, 22, 21, 20, 19, 18, 17, 16, 15, 14, 13, 12, 11, or below 10 kDa. In alternative embodiments, the MW is below 30, 29, 28, 27, 26, or 25 kDa.

The xylanase may also have a MW below 24000 Da. In particular embodiments, the MW is below 23000, 22000, 21000, 20000, 19000, 18000, 17000, 16000, 15000, 14000, 13000, 12000, 11000, or below 10000 Da. In alternative embodiments, the MW is below 30000, 29000, 28000, 27000, 26000, or 25000 Da.

In a particular embodiment, the indicated MW of the xylanase includes glycosylation, if any. In the alternative, the MW of the xylanase excludes glycosylation.

The MW may be determined by SDS-PAGE (Sodium Dodecyl Sulphate Poly Acryl Amide Gel Electrophoresis), which is a useful method, well known in the art, of determining molecular weight (MW) of proteins.

A suitable protocol for determining MW by SDS-PAGE is found in Example 3. In alternative embodiments, the Example 3 experiment is performed: (i) with a 10% Bis-Tris gel with MOPS running buffer; (ii) using the BenchMark Ladder (cat. no. 10747-012) commercially available from Invitrogen/Novex and which includes proteins at 20, 25 and 30kDa; and/or (iii) with Tricine and Tris-Glycine gels also available from Invitrogen/Novex. Example 3 is an SDS-PAGE of a fermentation supernatant, and there is no doubt which band represents the xylanase (only one major band; of the expected size). But in case there would be doubt, the xylanase might have to be purified to a higher extent, or, if you had an antibody you could do a western blot, or you could excise the bands in question and have an N-terminal sequence determined and this could identify the xylanase.

In the alternative, the MW of the xylanase may be calculated as the sum of the atomic masses of all the atoms of one molecule of the xylanase. To this end the average isotopic masses of amino acids in the mature protein and the average isotopic mass of one water molecule are used. The molecular weights may be derived from the 1997 IUPAC standard atomic weights. Programs for calculating MW of proteins are available, e.g. at the following internet site: http://www.expasy.org/tools/pi_tool.html (see also Gasteiger et al, in John M. Walker (ed): The Proteomics Protocols Handbook, Humana Press (2005), pp. 571-607).

In a still further alternative, the MW of the xylanase may be measured using mass spectrometry, e.g. Maldi-TOF, as is also well-known in the art.

Glycosylation is a phenomenon in which saccharides are attached to proteins. Glycosylation is only observed when expressing proteins in eukaryotes such as fungi and transgenic plants, but not in prokaryotes such as bacteria. There are various types of glycosylation: The N-linked glycosylation to the amide nitrogen of asparagine side chains, and the O-linked glycosylation to the hydroxy oxygen of serine and threonine side chains. E.g., mature glycoproteins may contain a variety of oligomannose N-linked oligosaccharides containing between 5 and 9 mannose residues.

Obviously, when a molecular weight is calculated on the basis of the protein sequence, it does not account for the effects of post-translational modifications such as glycosylation.

But glycosylation does effect protein migration in an SDS-PAGE gel, and it is also observed by mass spectrometry (Maldi-TOF). Therefore, if one wants to exclude the effect of glycosylation in these methods, the xylanase may be first deglycosylated. Deglycosylation kits are well known in the art and useful for this purpose, e.g. the Enzymatic CarboRelease™ Kit (cat. no. KE-DG01, which is commercially available from QA-Bio, LLC, 73 Sutton Place West, Palm Desert, CA 92211, US). This kit includes the enzymes, controls, and reagents required to remove all N-linked oligosaccharides and many O-linked sugars. The following deglycosylation enzymes are included in the kit: PNGase F (Chryseobacterium meningosepticum), O-Glycosidase (Streptococcus pneumoniae), Sialidase (Arthrobacter ureafaciens), beta-Galactosidase (Streptococcus pneumonia), Glucosaminidase (Streptococcus, pneumonia).

The molecular weight of a protein of course depends on the number as well as the exact chemical composition of its constituent amino acids. As an approximation of the MW, one may choose to refer only to the number of amino acids. Accordingly, the xylanase instead of having a limitation on its molecular weight may have a mature amino acid sequence consisting of below 220 amino acid residues in. In particular embodiments of this aspect, the number of amino acids is below 215, 210, 200, or below 195; preferably below 194, 193, 192, 191, or below 190; even more preferably below 189, 188, 187, 186, 185, 184, or below 183.

In particular embodiments, (i) the xylanase of the invention is used as the sole xylanase; (ii) the xylanase is not a 23 kDa GH11 xynA from Bacillus subtilis; (iii) the xylanase is not a mature part of the xylanase having the sequence of SWISSPROT:P18429; (iv) the xylanase is not a xylanase contained in the product Belfeed B 1100 MP or ML (commercially available from BelFeed, Belgium, see: http://www.agrimex.be).

### Examples

Chemicals used as buffers and substrates were commercial products of at least reagent grade.

### Example 1: In Vitro Test of Xylanases - Solubilization of NSP

The purpose of the current study was to investigate the efficacy of various xylanases as regards solubilization of non-starch polysaccharides (NSP).

### Xylanases

The following xylanases were tested:
The RONOZYME WX xylanase, a known monocomponent animal feed xylanase derived from Thermomyces lanuginosus and commercially available from DSM Nutritional Products, Wurmisweg 576, CH-4303 Kaiseraugst, Switzerland (this xylanase is also described in WO 96/23062);
A xylanase from Paenibacillus pabuli having the amino acid sequence of amino acids 1-182 of SEQ ID NO: 2 and described in WO 2005/079585;
A xylanase from Paenibacillus sp. (polymyxa) having the amino acid sequence of amino acids 1-184 of SEQ ID NO: 4 (UNIPROT:Q9F9B9);
A xylanase ("xyl II") from Aspergillus niger having the amino acid sequence of amino acids 1-188 of SEQ ID NO: 6 (very similar to the xylanase having SEQ ID NO: 6 in WO 97/13853); and
The mature part (excluding signal peptide and propeptide, if any) of another xylanase ("xyl III") from Aspergillus niger, the complete amino acid sequence of which is SEQ ID NO: 8 herein (identical to the xylanase having SEQ ID NO: 9 in WO 2004/018662).

The two bacterial xylanases were expressed in Bacillus subtilis, and the two Aspergillus xylanases were expressed in Aspergillus oryzae as is known in the art. The expression strains were fermented and the xylanase-containing supernatants used in the following experiments, except for the Paenibacillus pabuli xylanase which had been further purified using standard procedures. The enzyme protein content of the xylanase supernatants was estimated based on SDS-gels, whereas the enzyme protein content of the purified Paenibacillus pabuli xylanase was determined as described below.

The study was focused on quantification of the insoluble arabinoxylan content after in vitro incubation in a procedure mimicking the gastric and small intestinal digestion steps in monogastric digestion. In the in vitro system up to 60 test tubes, containing a substrate of interest, were incubated with HCl/pepsin (simulating gastric digestion), and subsequently with pancreatin (simulating intestinal digestion). Three test tubes were used for each treatment included. At the end of the intestinal incubation phase samples of the in vitro digesta were removed and analysed for insoluble NSP.

An outline of the in vitro procedure is shown in the below diagram in which pH and temperature indicate the respective set points (target values).

### Outline of in vitro digestion procedure

| **Components added** | **pH** | **Temperature** | **Time course** | **Simulated digestion phase** |
|---|---|---|---|---|
| 0.8 g substrate, 4.1 ml HCl (0.072 M) | 3.0 | 40°C | t=0 min | Mixing |
| 0.5 ml HCl (0.072 M) / pepsin (3000 U/g substrate), 0.1 ml enzyme solution | 3.0 | 40°C | t=30 min | Gastric digestion |
| 0.9 ml NaOH (0.215 M) | 6.8 | 40°C | t=1.5 hours | Intestinal digestion |
| 0.4 ml NaHCO₃(1M) / pancreatin (8 mg/g diet) | 6.8 | 40°C | t=2.0 hours | Intestinal digestion |
| Terminate incubation | 6.8 | 40°C | t=6.0 hours | |

### Conditions

| | |
|---|---|
| Substrate: | 0.35 g wheat, 0.21 g barley, 0.13 g soy bean meal, and 0.11 g wheat bran, provided as a premixed diet, which was milled to pass a 0.5 mm screen |
| pH: | stomach step = pH 3.0 / intestinal step = pH 6.8-7.0 |
| HCl: | 0.072 M for 1.5 hours (i.e. 30 min HCl-substrate premixing) |
| pepsin: | 3000 U /g diet for 1 hour (Sigma P-7000) |
| pancreatin: | 8 mg/g diet for 4 hours (Sigma P-7545) |
| Temperature: | 40°C |
| Replicates: | 3 |

### Solutions

0.215 M NaOH
0.072 M HCl
0.072 M HCl containing 6000 U pepsin per 5 ml
1 M NaHCO₃ containing 16 mg pancreatin per ml
100 mM NaAc-buffer, pH 5.0

### Enzyme protein determinations

The amount of xylanase enzyme protein (EP) is calculated on the basis of the A₂₈₀ values and the amino acid sequences (amino acid compositions) using the principles outlined in S.C.Gill & P.H. von Hippel, Analytical Biochemistry 182, 319-326, (1989).

### Experimental procedure for in vitro model

The experimental procedure was according to the above outline. pH was measured at time 1, 2.5, and 5.5 hours. Incubations were terminated after 6 hours and samples were removed and placed on ice before centrifugation (10000 x g, 10 min, 4°C). Supernatants were discarded and the pellet residue washed once with 100 mM acetate buffer (pH 5.0).

### Analysis

The analysis of residual NSP was made according to Theander et al (1995): Total dietary fiber determined as neutral sugar residues, uronic acid residues, and Klason lignin (the Uppsala method): Collaborative study, in J. AOAC Int. vol. 78, no. 4, pp. 1030-1044, except that cellulose was not analysed in the present example. In brief, the starch in the sample is removed by an enzyme digestion procedure with alpha-amylase and amyloglucosidase. The non-starch polysaccharides are then precipitated with 80% ethanol and hydrolysed at 125°C in 0.4 M sulphuric acid. Released neutral sugars are quantified by gas-liquid chromatography as alditol acetates, and their content calculated relative to an internal standard and taking the original sample weight into account.

Table 1 below shows the content (% of dry matter) of arabinose, xylose and arabinoxylan (sum of arabinose and xylose) residues in the feed after the in vitro incubation with the various xylanases. The control is without added xylanase.

**Table 1**

| | Enzyme Dosage (mg EP/kg diet) | | | | | |
|---|---|---|---|---|---|---|
| Xylanase Sample | 0 | 12.5 | 12.5 | 12.5 | 12.5 | 12.5 |
| | Contro l | RONOZYME WX | P. pabuli | P. polymyxa | A. niger xyl II | A. niger xyl III |
| Arabinose residues | 2.67^{a} | 2.32^{bd} | 2.11^{bc} | 2.18b^{cd} | 2.35^{d} | 2.68^{a} |
| Standard deviation | 0.20 | 0.11 | 1.04 | 0.11 | 0.15 | 0.08 |
| Relative reduction | 100 | 87 | 79 | 82 | 88 | 100 |
| Xylose residues | 4.54^{a} | 3.48^{b} | 2.93^{c} | 2.98^{c} | 3.82^{d} | 4.56^{a} |
| Standard deviation | 0.27 | 0.16 | 0.05 | 0.14 | 0.22 | 0.20 |
| Relative reduction | 100 | 77 | 65 | 66 | 84 | 100 |
| Insoluble Arabinoxyla n | 7.21^{a} | 5.80^{b} | 5.04^{c} | 5.16^{c} | 6.16^{bd} | 7.25^{a} |
| Standard deviation | 0.46 | 0.27 | 0.09 | 0.25 | 0.38 | 0.28 |
| Relative reduction | 100 | 80 | 70 | 72 | 85 | 100 |

| | | | | | | |
|---|---|---|---|---|---|---|
| abcd: Means within a row not sharing a common letter superscript differ with statistical significance (P<0.05). | | | | | | |

It appears from Table 1 that, surprisingly, the P. pabuli and P. polymyxa xylanases are statistically significantly better when it comes to solubilization of insoluble fibre polysaccharides (NSP) as compared to 1) the control without added xylanase, 2) the known animal feed xylanase of RONOZYME WX, as well as 3) the two A. niger xylanases.

### Example 2: Dose Response Effect

The Paenibacillus pabuli xylanase was tested in various dosages in an in vitro experiment as described in Example 1.

Table 2 below shows the content (% of fresh weight) of insoluble arabinose, xylose and arabinoxylan (sum of arabinose and xylose) residues in the feed after the in vitro incubation with this xylanase in various dosages. The control is without added xylanase.

**Table 2**

| Sample | Control | P. pabuli xylanase | | |
|---|---|---|---|---|
| Enzyme dosage (mg EP/kg diet) | 0 | 0.7 | 7.0 | 70 |
| Arabinose residues | 1.99^{a} | 1.91^{ab} | 1.82^{b} | 1.68^{c} |
| Standard deviation | 0.018 | 0.043 | 0.084 | 0.053 |
| Relative reduction | 100 | 96 | 91 | 84 |
| Xylose residues | 3.26^{a} | 3.00^{b} | 2.64^{c} | 2.05^{d} |
| Standard deviation | 0.065 | 0.062 | 0.144 | 0.066 |
| Relative reduction | 100 | 92 | 81 | 63 |
| Arabinoxylan | 5.25^{a} | 4.91^{b} | 4.46^{c} | 3.73^{d} |
| Standard deviation | 0.082 | 0.104 | 0.228 | 0.119 |
| Relative reduction | 100 | 94 | 85 | 71 |

| | | | | |
|---|---|---|---|---|
| abcd: Means within a row not sharing a common letter superscript differ with statistical significance (P<0.05). | | | | |

It appears from Table 2 that, there is a clear and statistically significant dose-response effect of the Paenibacillus pabuli xylanase on solubilization of insoluble fibre polysaccharides (NSP).

### Example 3: Determination of Molecular Weight

A transformed Aspergillus oryzae host expressing the xylanase of amino acids 1-188 of SEQ ID NO: 6 was fermented for four days in 500 ml baffled shake flasks with 100 ml YP+2%G medium (10g yeast extract, 20g peptone, water to 1L, autoclave at 121°C, 20 minutes, add 100ml 20% sterile glucose solution) at 30°C and 200 RPM. The fermentation liquor was filtered through a 0.22 um (micrometer) filter unit to provide a supernatant.

10 ul (microliter) of the supernatant was mixed with 10ul NuPAGE® LDS sample buffer (4X) (available from Invitrogen, cat. no. NP0007), 2ul 1% EDTA, 2ul 6% PMSF, 4ul 0.5M DTT, and 2 ul H₂O, to a total volume of 20ul.

The 20 ul sample was heated to 99°C for 3 minutes and applied to an SDS-PAGE gel of the type NuPAGE® Novex 10 % Bis-Tris 1 mm Gels, available from Invitrogen (cat. no. NP0301 BOX).
Running buffer: Upper buffer chamber, 200ml 1 X NuPAGE® MES SDS running buffer (cat. no. NP0002) containing 500ul NuPAGE® antioxidant (cat. no. NP0005). Lower buffer chamber, 600ml 1 X NuPAGE® MES SDS running buffer.
Run conditions: Two steps, viz. 30min 50mAmp, and 20 min 100mAmp.
Stain: Simply Blue Stain^{™} Safe stain from Invitrogen (cat. no. LC6065).
Rinse: 3 times for 5 min in de-ionizied water, approximately 100ml for each time.
Stain: Cover the gel with Simply Blue stain solution. Stain for at least one hour at room temperature.
Destain: Discard the stain and wash the gel in deionizied water.
MW marker: Amersham's Low Molecular Weight Calibration Kit for SDS Electrophoresis (product code 17-0446-01).

From the SDS-PAGE gel, the molecular weight of the xyl II Aspergillus niger xylanase was judged to below 24 kDa.

### Example 4: Determination of Xylanase Activity

This assay is an example of a xylanase assay. It is particularly suitable for determining the activity of the Paenibacillus xylanases of the present invention.
Substrate: 0.2% AZCL-Arabinoxylan from wheat (Megazyme) in 0.2 M Na-phosphate buffer pH 6.0 + 0.01% Triton-x-100.
Standard: Bio-Feed Wheat FXU standard (such as batch 43-1195, which is available on request from Novozymes A/S, Krogshoejvej 36, DK-2880 Bagsvaerd, Denmark).
Dilution: In 0.01% Triton-x-100.
FXU/ml: 0.05; 0.10; 0.15; 0.20; 0.25; 0.30; 0.40.
Method: 900 ul (microliter) substrate is preheated to 37°C in a thermomixer . 100 ul sample is added. Incubate for 15 min at 37°C at maximum speed. On ice for 2 min. Spin 1 min 20000 x G. 2 x 200 ul supernatant is transferred to a micro titter plate. Endpoint OD 590 nm is measured.

### Example 5: Solubilization and Total Degradation of NSP in Vitro

The purpose of the current study was to compare the efficacy of a xylanase of the invention with a homologous, known animal feed xylanase as regards solubilization and total degradation of non-starch polysaccharides (NSP).

### Xylanases

The following xylanases were tested:
The RONOZYME WX xylanase and a xylanase of the invention from Paenibacillus pabuli (both described in Example 1), and a comparative xylanase having the sequence of amino acids 1-185 of SEQ ID NO: 9 (Swissprot Q6TLP3).

The comparative xylanase was expressed from a synthetic gene in a protease-weak strain of Bacillus subtilis. A xylanase-containing culture broth was prepared by fermentation thereof, and the xylanase was purified using standard procedures. To inactivate possible proteases, the filtered culture broth, added the same volume of 50mM acetic acid pH 4.0 and adjusted to pH 4.0 with 50% acetic acid, was incubated for 30minutes at 37°C in 500 mL shakeflasks containing 250 mL in a waterbath. The solution was mixed gently with a magnetic stirrer during the incubation. After incubation, the solution was centrifuged for 30minutes at 12,000g and the supernatant was separated from the pellet. The protease activity was measured as follows: Substrate N-Succinyl-Ala-Ala-Pro-Phe-pNitroanilid (Sigma, S7388), assay buffer: 100mM HEPES pH7.5 (0.01% Triton X-100), enzyme dilutions in 0.01% Triton X-100, and reading OD405 after 10 minutes at 25°C.

The resulting xylanase was substantially pure (one band on an SDS-gel) and had a moleular weight of approximately 22kDa (by SDS-PAGE).

The enzyme protein content of the commercial xylanase was estimated based on SDS-gels, whereas the enzyme protein content of the purified Paenibacillus pabuli xylanase and the comparative xylanase was determined as described in Example 1.

### Experimental procedure for in vitro model

The study was focused on quantification of insoluble as well as total arabinoxylan content after in vitro incubation in a monogastric digestion procedure as described in Example 1 (See: Outline of in vitro digestion procedure, Conditions, Solutions, and Enzyme protein determinations).

For determination of insoluble arabinoxylans, pH was measured at time 1, 2.5, and 5.5 hours. Incubations were terminated after 6 hours and samples were removed and placed on ice before centrifugation (10000 x g, 10 min, 4°C). Supernatants were discarded and the insoluble pellet residue was washed once with acetate buffer (pH 5.0 and 100 mM).

For determination of total arabinoxylans, pH was measured at time 1, 2.5, and 5.5 hours. Incubations were terminated after 6 hours. Absolute ethanol was added to obtain a concentration of 80% ethanol in the sample in order to precipitate all polysaccharides of a degree of polymerization (DP) greater than 10 (DP>10). Samples were then cooled (4°C) on ice before centrifugation (10000 x g, 10 min, 4°C). Supernatants were discarded and the pellet residue washed once with 80% ethanol.

### Analysis

The analysis of arabinoxylan NSP in the pellet residue was performed according to Theander et al, as described in Example 1. Polysaccharides (DP>10) are hydrolysed in sulphuric acid together with an internal standard (Myo-inositol) and released neutral sugars (arabinose+xylose) quantified.

### Results

Table 3 shows the dry matter content (%) of insoluble arabinose+xylose (arabinoxylan) residues in the feed, i.e. arabinoxylan NSP which is insoluble after the in vitro incubation with the xylanases.

Table 4 shows the dry matter content (%) of total arabinose+xylose (arabinoxylan) residues in the feed, i.e. the sum of insoluble and soluble arabinoxylan NSP.

In both Tables the control is without added xylanase.

**Table 3**

| Xylanase Sample | Enzyme Dosage (mg EP/kg diet) | | | | | |
|---|---|---|---|---|---|---|
| | 0 | 5 | 5 | 20 | 5 | 20 |
| | Control | RONOZYME WX | P. pabuli | P. pabuli | Q6TLP3 | Q6TLP 3 |
| Insoluble Arabinoxylan | 7.34^{a} | 6.89^{b} | 5.78^{de} | 5.51^{e} | 6.36^{c} | 6.00^{cd} |
| Standard deviation | 0.42 | 0.076 | 0.22 | 0.20 | 0.05 | 0.14 |
| Relative reduction | 100 | 94 | 79 | 75 | 87 | 82 |

| | | | | | | |
|---|---|---|---|---|---|---|
| abcde: Means within a row not sharing a common letter superscript differ with statistical significance (P<0.05). | | | | | | |

**Table 4**

| Xylanase Sample | Enzyme Dosage (mg EP/kg diet) | | | | | |
|---|---|---|---|---|---|---|
| | 0 | 5 | 5 | 20 | 5 | 20 |
| | Control | RONOZYME WX | P. pabuli | P. pabuli | Q6TLP3 | Q6TLP 3 |
| Total Arabinoxylan | 7.94^{ab} | 8.17^{a} | 7.48^{cd} | 7.32^{d} | 7.82^{abc} | 7.57^{bcd} |
| Standard deviation | 0.16 | 0.26 | 0.072 | 0.25 | 0.31 | 0.11 |
| Relative reduction | 100 | 103 | 94 | 92 | 98 | 95 |

| | | | | | | |
|---|---|---|---|---|---|---|
| abcd: Means within a row not sharing a common letter superscript differ with statistical significance (P<0.05). | | | | | | |

It appears from Table 3 that, surprisingly, the P. pabuli xylanase is statistically significantly better regarding the capacity to solubilize the arabinoxylan fraction as compared to 1) the control without added xylanase, 2) the commercial animal feed xylanase of RONOZYME WX, as well as 3) the comparative Q6TLP3 xylanase.

The content of soluble arabinoxylans will go into the supernatant after centrifugation of the in vitro incubation mixtures, and will therefore not be included in the determination of insoluble arabinoxylans.

The content of total arabinoxylan (Table 4) includes the content of insoluble arabinoxylans as well as the content of soluble arabinoxylans.

The differences between corresponding Table 4 and Table 3 values are indicative of the amount of NSP which has been degraded to oligomers smaller than DP 10 by the xylanases during the in vitro incubation.

Clearly, the xylanases investigated are more efficient in the solubilization of the arabinoxylan fraction (Table 3) than they are in the total degradation (Table 4). This is a typical trait of family 11 xylanases. Still it appears from Table 4 that the P. pabuli xylanase at 5 mg EP/kg diet is also significantly better regarding the capacity to degrade the arabinoxylan fraction as compared to 1) the control without added xylanase, and 2) the known animal feed xylanase of RONOZYME WX, and it is 3) numerically more efficient (4%) than the comparative Q6TLP3 xylanase.

### Example 6: Animal Feed and Feed Additive Compositions

A formulation of the Paenibacillus pabuli xylanase of SEQ ID NO: 2 containing 0.050 g xylanase enzyme protein is added to the following premix (per kilo of premix):

| | | |
|---|---|---|
| 5000000 | IE | Vitamin A |
| 1000000 | IE | Vitamin D3 |
| 13333 | mg | Vitamin E |
| 1000 | mg | Vitamin K3 |
| 750 | mg | Vitamin B1 |
| 2500 | mg | Vitamin B2 |
| 1500 | mg | Vitamin B6 |
| 7666 | mcg | Vitamin B12 |
| 12333 | mg | Niacin |
| 33333 | mcg | Biotin |
| 300 | mg | Folic Acid |
| 3000 | mg | Ca-D-Panthothenate |
| 1666 | mg | Cu |
| 16666 | mg | Fe |
| 16666 | mg | Zn |
| 23333 | mg | Mn |
| 133 | mg | Co |
| 66 | mg | I |
| 66 | mg | Se |
| 5.8 | % | Calcium |
| 25 | % | Sodium |

### Animal Feed

This is an example of an animal feed (broiler feed) comprising 0.5 mg/kg (0.5 ppm) of the Paenibacillus pabuli xylanase of SEQ ID NO: 2 (calculated as xylanase enzyme protein):
65.00 % wheat
32.35% Soybean meal (50% crude protein, CP)
1.0% Soybean oil
0.2 %DL-Methionine
0.22% DCP (dicalcium phosphate)
0.76% CaCO₃ (calcium carbonate)
0.32% Sand
0.15% NaCl (sodium chloride)
1 % of the above Premix

The ingredients are mixed, and the feed is pelleted at the desired temperature, e.g. 70°C.

## Claims

1. Use in animal feed of a xylanase having a percentage of identity to amino acids 1-182 of SEQ ID NO: 2 of at least 90%, the percentage of identity being determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage.

2. Use according to claim 1 of a xylanase as defined in claim 1 in the preparation of a composition for use in animal feed.

3. Use according to claim 1 of a xylanase as defined in claim 1 for the solubilization of non-starch polysaccharides during gastric and intestinal digestion.

4. Use according to claim 1 of a xylanase as defined in claim 1 for the degradation of non-starch polysaccharides during gastric and intestinal digestion.

5. Use according to claim 1 of a xylanase as defined in claim 1 for pre-treatment of animal feed or animal feed components.

6. An animal feed composition comprising a xylanase as defined in claim 1, and
(a) at least one fat soluble vitamin;
(b) at least one water soluble vitamin; and/or
(c) at least one trace mineral.

7. The animal feed composition of claim 6 further comprising at least one enzyme selected from the following group of enzymes: Another xylanase, and/or beta-glucanase.

8. The animal feed composition of any one of claims 6-7 which is an animal feed additive.

9. The animal feed composition of claims 6 to 8 having a crude protein content of 50 to 800 g/kg and comprising a xylanase as defined in claim 1.

10. A method for improving the nutritional value of an animal feed, wherein a xylanase as defined in claim 1, or a composition of any one of claims 6-8 is added to the feed.

## Patentansprüche

1. Verwendung in Tierfutter einer Xylanase mit einem Prozentsatz an Identität zu Aminosäuren 1-182 von SEQ ID NO: 2 von mindestens 90%, wobei der Prozentsatz an Identität bestimmt wird durch i) Abgleichen der zwei Aminosäuresequenzen unter Verwendung des Needle-Programms, mit der BLOSUM62-Substitutionsmatrix, einem Lückenöffnungsstrafwert (gap opening penalty) von 10, und einem Lückenerweiterungsstrafwert (gap extension penalty) von 0,5; ii) Zählen der Anzahl exakter Übereinstimmungen im Abgleich; iii) Dividieren der Anzahl exakter Übereinstimmungen durch die Länge der kürzesten der zwei Aminosäuresequenzen, und iv) Umwandeln des Ergebnisses der Division von iii) in einen Prozentsatz.

2. Verwendung nach Anspruch 1 einer wie in Anspruch 1 definierten Xylanase in der Zubereitung einer Zusammensetzung zur Verwendung in Tierfutter.

3. Verwendung nach Anspruch 1 einer wie in Anspruch 1 definierten Xylanase zum Löslichmachen von Nicht-Stärke-Polysacchariden während der Verdauung in Magen und Darm.

4. Verwendung nach Anspruch 1 einer wie in Anspruch 1 definierten Xylanase zum Abbau von Nicht-Stärke-Polysacchariden während der Verdauung in Magen und Darm.

5. Verwendung nach Anspruch 1 einer wie in Anspruch 1 definierten Xylanase zur Vorbehandlung von Tierfutter oder Tierfutterbestandteilen.

6. Tierfutterzusammensetzung umfassend eine wie in Anspruch 1 definierte Xylanase, und
(a) mindestens ein fettlösliches Vitamin;
(b) mindestens ein waserlösliches Vitamin; und/oder
(c) mindestens ein Spurenmineral.

7. Tierfutterzusammensetzung nach Anspruch 6, weiterhin umfassend mindestens ein Enzym ausgewählt aus der folgenden Gruppe an Enzymen: eine andere Xylanase und/oder beta-Glucanase.

8. Tierfutterzusammensetzung nach einem beliebigen der Ansprüche 6-7, die ein Tierfutterzusatz ist.

9. Tierfutterzusammensetzung nach Ansprüchen 6 bis 8 mit einem Rohproteingehalt von 50 bis 800 g/kg und umfassend eine wie in Anspruch 1 definierte Xylanase.

10. Verfahren zum Verbessern des Nährwertes eines Tierfutters, wobei eine wie in Anspruch 1 definierte Xylanase oder eine Zusammensetzung gemäß einem beliebigen der Ansprüche 6-8 zum Futter zugefügt wird.

## Revendications

1. Utilisation dans des aliments pour animaux d'une xylanase présentant un pourcentage d'identité avec les acides aminés 1 à 182 de SEQ ID NO : 2 d'au moins 90 %, le pourcentage d'identité étant déterminé par i) l'alignement des deux séquences d'acides aminés en utilisant le programme Needle, avec la matrice de substitution BLOSUM62, une pénalité d'ouverture de brèche de 10, et une pénalité d'extension de brèche de 0,5 ; ii) le comptage du nombre de correspondances exactes dans l'alignement ; iii) la division du nombre de correspondances exactes par la longueur de la plus courte des deux séquences d'acides aminés, et iv) la conversion du résultat de la division de iii) en pourcentage.

2. Utilisation selon la revendication 1 d'une xylanase telle que définie dans la revendication 1 dans la préparation d'une composition pour une utilisation dans des aliments pour animaux.

3. Utilisation selon la revendication 1 d'une xylanase telle que définie dans la revendication 1 pour la solubilisation des polysaccharides non amidon durant la digestion gastrique et intestinale.

4. Utilisation selon la revendication 1 d'une xylanase telle que définie dans la revendication 1 pour la dégradation des polysaccharides non amidon durant la digestion gastrique et intestinale.

5. Utilisation selon la revendication 1 d'une xylanase telle que définie dans la revendication 1 pour le prétraitement d'aliments pour animaux ou de composants d'aliments pour animaux.

6. Composition d'aliment pour animaux comprenant une xylanase telle que définie dans la revendication 1, et
(a) au moins une vitamine liposoluble ;
(b) au moins une vitamine hydrosoluble ; et/ou
(c) au moins un oligo-élément.

7. Composition d'aliment pour animaux selon la revendication 6 comprenant en outre au moins une enzyme choisie dans le groupe suivant d'enzymes ; une autre xylanase et/ou une bêta-glucanase.

8. Composition d'aliment pour animaux selon l'une quelconque des revendications 6 ou 7 qui est un additif d'aliments pour animaux.

9. Composition d'aliment pour animaux selon les revendications 6 à 8 ayant une teneur en protéines brutes de 50 à 800 g/kg et comprenant une xylanase telle que définie dans la revendication 1.

10. Méthode d'amélioration de la valeur nutritionnelle d'un aliment pour animaux, dans laquelle une xylanase telle que définie dans la revendication 1, ou une composition selon l'une quelconque des revendications 6 à 8 est ajoutée à l'aliment.


### SEQUENCE LISTING

<110> Novozymes A/S
<120> xylanases for Animal Feed
<130> 10987.204-WO
<160> 9
<170> PatentIn version 3.4
<210> 1
   <211> 630
   <212> DNA
   <213> Paenibacillus pabuli DSM 16232
<220>
   <221> CDS
   <222> (1)..(630)
<220>
   <221> sig_peptide
   <222> (1)..(84)
<220>
   <221> mat_peptide
   <222> (85)..(630)
<400> 1
   atg ttt aaa ttc gga aaa aaa ttg tta act gtt gtc ctt gcc gct tcc 48
   Met Phe Lys Phe Gly Lys Lys Leu Leu Thr Val Val Leu Ala Ala Ser
   -25 -20 -15
   atg agt ttt ggt gta ttc gcc gct acg aca ggt gct aca gat tac tgg 96
   Met Ser Phe Gly Val Phe Ala Ala Thr Thr Gly Ala Thr Asp Tyr Trp
   -10 -5 -1 1
   cag aac tgg aca gat ggc ggg ggt act gtt aat gcc gtg aac ggt tcg 144
   Gln Asn Trp Thr Asp Gly Gly Gly Thr Val Asn Ala Val Asn Gly Ser
   5 10 15 20
   gga gga aac tac agt gta aac tgg cag aac acg ggg aac ttt gtt gtc 192
   Gly Gly Asn Tyr Ser Val Asn Trp Gln Asn Thr Gly Asn Phe Val Val
   25 30 35
   ggt aaa ggg tgg act tac ggt aca cct aat cgt gta gtg aat tac aat 240
   Gly Lys Gly Trp Thr Tyr Gly Thr Pro Asn Arg Val Val Asn Tyr Asn
   40 45 50
   gcg ggt gta ttc tct cca tcc ggc aac gga tat ttg acg ttt tac ggg 288
   Ala Gly Val Phe Ser Pro Ser Gly Asn Gly Tyr Leu Thr Phe Tyr Gly
   55 60 65
   tgg aca cgg aat gca ctt att gaa tac tac gtg gtg gat aac tgg gga 336
   Trp Thr Arg Asn Ala Leu Ile Glu Tyr Tyr Val Val Asp Asn Trp Gly
   70 75 80
   aca tac cgg cca acc gga aca tac aaa ggc aca gta acc agt gat ggt 384
   Thr Tyr Arg Pro Thr Gly Thr Tyr Lys Gly Thr Val Thr Ser Asp Gly
   85 90 95 100
   ggc aca tat gac atc tat act acg atg aga tac aat cag cca tcc att 432
   Gly Thr Tyr Asp Ile Tyr Thr Thr Met Arg Tyr Asn Gln Pro Ser Ile
   105 110 115
   gac ggg tat tca aca ttc ccg caa tac tgg agt gtt aga caa tcc aaa 480
   Asp Gly Tyr Ser Thr Phe Pro Gln Tyr Trp Ser Val Arg Gln Ser Lys
   120 125 130
   cgt cca atc ggt gta aat tcc caa att acg ttc cag aat cac gta aat 528
   Arg Pro Ile Gly Val Asn Ser Gln Ile Thr Phe Gln Asn His Val Asn
   135 140 145
   gcg tgg gcg agc aag ggc atg tac ttg ggt aac agc tgg tcc tat caa 576
   Ala Trp Ala Ser Lys Gly Met Tyr Leu Gly Asn Ser Trp Ser Tyr Gln
   150 155 160
   gtg atg gcc acc gaa gga tat caa agt agc ggt agt tcg aat gtg act 624
   Val Met Ala Thr Glu Gly Tyr Gln Ser Ser Gly Ser Ser Asn Val Thr
   165 170 175 180
   gtt tgg 630
   Val Trp
<210> 2
   <211> 210
   <212> PRT
   <213> Paenibacillus pabuli DSM 16232
<400> 2
   Met Phe Lys Phe Gly Lys Lys Leu Leu Thr Val Val Leu Ala Ala Ser
   -25 -20 -15
   Met Ser Phe Gly Val Phe Ala Ala Thr Thr Gly Ala Thr Asp Tyr Trp
   -10 -5 -1 1
   Gln Asn Trp Thr Asp Gly Gly Gly Thr Val Asn Ala Val Asn Gly Ser
   5 10 15 20
   Gly Gly Asn Tyr Ser Val Asn Trp Gln Asn Thr Gly Asn Phe Val Val
   25 30 35
   Gly Lys Gly Trp Thr Tyr Gly Thr Pro Asn Arg Val Val Asn Tyr Asn
   40 45 50
   Ala Gly Val Phe Ser Pro Ser Gly Asn Gly Tyr Leu Thr Phe Tyr Gly
   55 60 65
   Trp Thr Arg Asn Ala Leu Ile Glu Tyr Tyr Val Val Asp Asn Trp Gly
   70 75 80
   Thr Tyr Arg Pro Thr Gly Thr Tyr Lys Gly Thr Val Thr Ser Asp Gly
   85 90 95 100
   Gly Thr Tyr Asp Ile Tyr Thr Thr Met Arg Tyr Asn Gln Pro Ser Ile
   105 110 115
   Asp Gly Tyr Ser Thr Phe Pro Gln Tyr Trp Ser Val Arg Gln Ser Lys
   120 125 130
   Arg Pro Ile Gly Val Asn Ser Gln Ile Thr Phe Gln Asn His Val Asn
   135 140 145
   Ala Trp Ala Ser Lys Gly Met Tyr Leu Gly Asn Ser Trp Ser Tyr Gln
   150 155 160
   Val Met Ala Thr Glu Gly Tyr Gln Ser Ser Gly Ser Ser Asn Val Thr
   165 170 175 180
   Val Trp
<210> 3
   <211> 1409
   <212> DNA
   <213> Paenibacillus sp. KCTC 8848P
<220>
   <221> CDS
   <222> (725)..(1360)
<220>
   <221> sig_peptide
   <222> (725)..(808)
<220>
   <221> mat_peptide
   <222> (809)..(1360)
<400> 3
   attacccgta ataaaatggt aatccagtaa tatcagtcca gttggccgag ctcatcttga      60
   ttcaatttta actaattggc aagcattgag tgacacatga aatccgggta ttttgtcttc     120
   agattctctc tcatttctta tctctttcga acaataacac ttttgttgat ccagttaact     180
   gtgtcttcat gaaacgtgtg atagcacctg tacctgaccc gagctctgcg acggccttta     240
   cctcacccca gggtacagat tcaaccacct tagctgccag aaatcgagaa ctaggcataa     300
   cgcttcccac tcgttgtgga ttggtgagga atccttgcaa aaaaagtagg tattctttga     360
   aattcaaccg tttatccccc atgacttatt ttcttccttt tgttttttgt tcatataaaa      420
   aacagcagcg aaaaaatcta aggattttac gaggatatat ctaaagaaaa tctgaagaac     480
   tctaaagaag aaactcaata aactcccgac ggatatatat aacttaggga aatctaaaca     540
   tagtaaagtg taatatttaa tttgcaaagt ggttatgtgc tcgttattat tatataattt     600
   tacatataaa ggaggtgaaa gtaagaaaac acgggatatg gttcacatga gtgatgcact     660
   gcaacggaga atcttccgac aagcaggtgt agaacgaaaa tcacaaaatt ttaggaggca     720
   aatt atg ttt aaa agt agt aag aaa ctg tta acg gta gtt ctt gca gct      769
        Met Phe Lys Ser Ser Lys Lys Leu Leu Thr Val Val Leu Ala Ala
                    -25                 -20                 -15
   tcc atg agt ttt ggt ttt ttt gca tca acc tca aat gca gcg acg gac       817
   Ser Met Ser Phe Gly Phe Phe Ala Ser Thr Ser Asn Ala Ala Thr Asp
               -10                 -5                  -1  1
   tac tgg caa aat tgg acc gat ggc ggt ggg acg gtt aat gct gtt aat       865
   Tyr Trp Gln Asn Trp Thr Asp Gly Gly Gly Thr Val Asn Ala Val Asn
       5                   10                  15
   ggg tcc ggc ggc aat tac agc gta aca tgg aaa aat agc ggg aat ttt       913
   Gly Ser Gly Gly Asn Tyr Ser Val Thr Trp Lys Asn Ser Gly Asn Phe
   20                  25                  30                  35
   gtt gtc ggc aaa ggc tgg act act gga tcg cca gac aga acg att aat       961
   Val Val Gly Lys Gly Trp Thr Thr Gly Ser Pro Asp Arg Thr Ile Asn
                   40                  45                  50
   tac aat gcc ggt gtc tgg gcg ccg tcc ggt aat gga tat ttg gcc ctc      1009
   Tyr Asn Ala Gly Val Trp Ala Pro Ser Gly Asn Gly Tyr Leu Ala Leu
               55                  60                  65
   tac ggg tgg acg aga aac tca ctc atc gaa tat tac gtt gtt gat agc      1057
   Tyr Gly Trp Thr Arg Asn Ser Leu Ile Glu Tyr Tyr Val Val Asp Ser
           70                  75                  80
   tgg ggg act tat cga cct acc gga acg tat aaa ggt acg gtg acc agt      1105
   Trp Gly Thr Tyr Arg Pro Thr Gly Thr Tyr Lys Gly Thr Val Thr Ser
       85                  90                  95
   gat ggg ggt aca tat gac atc tac aca aca atg cga tac gac gca cct      1153
   Asp Gly Gly Thr Tyr Asp Ile Tyr Thr Thr Met Arg Tyr Asp Ala Pro
   100                 105                 110                 115
   tcc att gaa ggc caa aaa acg aca ttt atc cag tac tgg agt gtt cga      1201
   Ser Ile Glu Gly Gln Lys Thr Thr Phe Ile Gln Tyr Trp Ser Val Arg
                   120                 125                 130
   cag acg aag aga ccg acc ggg ggc aac tcc acg atc act ttc agc aat      1249
   Gln Thr Lys Arg Pro Thr Gly Gly Asn Ser Thr Ile Thr Phe Ser Asn
               135                 140                 145
   cac gtg aag gct tgg gcg agg caa gga atg cat ctg ggg aac aac tgg      1297
   His Val Lys Ala Trp Ala Arg Gln Gly Met His Leu Gly Asn Asn Trp
           150                 155                 160
   tct tac cag gtg tta gcg aca gag ggg tat cag agt agc ggg agc tct      1345
   Ser Tyr Gln Val Leu Ala Thr Glu Gly Tyr Gln Ser Ser Gly Ser Ser
       165                 170                 175
   aac gta acg gtg tgg taaccatgaa ggcttaaagc cgtattacaa catagataag      1400
   Asn Val Thr Val Trp
   180
   gaaaagaaa                                                            1409
<210> 4
   <211> 212
   <212> PRT
   <213> Paenibacillus sp. KCTC 8848P
<400> 4
   Met Phe Lys Ser Ser Lys Lys Leu Leu Thr Val Val Leu Ala Ala Ser
               -25                 -20                 -15
   Met Ser Phe Gly Phe Phe Ala Ser Thr Ser Asn Ala Ala Thr Asp Tyr
           -10                 -5                  -1  1
   Trp Gln Asn Trp Thr Asp Gly Gly Gly Thr Val Asn Ala Val Asn Gly
   5                   10                  15                  20
   Ser Gly Gly Asn Tyr Ser Val Thr Trp Lys Asn Ser Gly Asn Phe Val
                   25                  30                  35
   Val Gly Lys Gly Trp Thr Thr Gly Ser Pro Asp Arg Thr Ile Asn Tyr
               40                  45                  50
   Asn Ala Gly Val Trp Ala Pro Ser Gly Asn Gly Tyr Leu Ala Leu Tyr
           55                  60                  65
   Gly Trp Thr Arg Asn Ser Leu Ile Glu Tyr Tyr Val Val Asp Ser Trp
       70                  75                  80
   Gly Thr Tyr Arg Pro Thr Gly Thr Tyr Lys Gly Thr Val Thr Ser Asp
   85                  90                  95                  100
   Gly Gly Thr Tyr Asp Ile Tyr Thr Thr Met Arg Tyr Asp Ala Pro Ser
                   105                 110                 115
   Ile Glu Gly Gln Lys Thr Thr Phe Ile Gln Tyr Trp Ser Val Arg Gln
               120                 125                 130
   Thr Lys Arg Pro Thr Gly Gly Asn Ser Thr Ile Thr Phe Ser Asn His
           135                 140                 145
   Val Lys Ala Trp Ala Arg Gln Gly Met His Leu Gly Asn Asn Trp Ser
       150                 155                 160
   Tyr Gln Val Leu Ala Thr Glu Gly Tyr Gln Ser Ser Gly Ser Ser Asn
   165                 170                 175                 180
   Val Thr Val Trp
<210> 5
   <211> 786
   <212> DNA
   <213> Aspergillus niger DSM 12665
<220>
   <221> CDS
   <222> (6)..(680)
<220>
   <221> sig_peptide
   <222> (6)..(116)
<220>
   <221> mat_peptide
   <222> (117)..(680)
<400> 5
   caatc atg ctc acc aag aac ctt ctc ctc tgc ttt gcc gcg gct aag gct     50
         Met Leu Thr Lys Asn Leu Leu Leu Cys Phe Ala Ala Ala Lys Ala
                 -35                 -30                 -25
   gct ctg gct gtt ccc cac gac tct gtc gcc cag cgt tcg gat gcc ttg       98
   Ala Leu Ala Val Pro His Asp Ser Val Ala Gln Arg Ser Asp Ala Leu
           -20                 -15                 -10
   cac atg ctc tct gag cgc tcg acc ccg agc tcg acc ggc gag aac aac      146
   His Met Leu Ser Glu Arg Ser Thr Pro Ser Ser Thr Gly Glu Asn Asn
       -5                  -1  1               5                   10
   ggc ttc tac tac tcc ttc tgg acc gac ggc ggt ggc gac gtg acc tac      194
   Gly Phe Tyr Tyr Ser Phe Trp Thr Asp Gly Gly Gly Asp Val Thr Tyr
                   15                  20                  25
   acc aac gga gat gct ggt gcc tac act gtt gag tgg tcc aac gtg ggc      242
   Thr Asn Gly Asp Ala Gly Ala Tyr Thr Val Glu Trp Ser Asn Val Gly
               30                  35                  40
   aac ttt gtc ggt gga aag ggc tgg aac ccc gga agt gcg cag gac atc      290
   Asn Phe Val Gly Gly Lys Gly Trp Asn Pro Gly Ser Ala Gln Asp Ile
           45                  50                  55
   acc tac agc ggc acc ttc acc cct agc ggc aac ggc tat ctc tcc gtc      338
   Thr Tyr Ser Gly Thr Phe Thr Pro Ser Gly Asn Gly Tyr Leu Ser Val
       60                  65                  70
   tat ggc tgg acc act gac ccc ctg atc gag tac tac atc gtc gag tcc      386
   Tyr Gly Trp Thr Thr Asp Pro Leu Ile Glu Tyr Tyr Ile Val Glu Ser
   75                  80                  85                  90
   tac ggc gac tac aac ccc ggc agt gga ggc aca tac aag ggc acc gtc      434
   Tyr Gly Asp Tyr Asn Pro Gly Ser Gly Gly Thr Tyr Lys Gly Thr Val
                   95                  100                 105
   acc tcg gac gga tcc gtt tac gat atc tac acg gct acc cgt acc aat      482
   Thr Ser Asp Gly Ser Val Tyr Asp Ile Tyr Thr Ala Thr Arg Thr Asn
               110                 115                 120
   gct gct tcc att cag gga acc gct acc ttc act cag tac tgg tcc gtc      530
   Ala Ala Ser Ile Gln Gly Thr Ala Thr Phe Thr Gln Tyr Trp Ser Val
           125                 130                 135
   cgc cag aac aag aga gtt ggc gga act gtt acc acc tcc aac cac ttc      578
   Arg Gln Asn Lys Arg Val Gly Gly Thr Val Thr Thr Ser Asn His Phe
       140                 145                 150
   aat gct tgg gct aag ctg gga atg aac ctg ggt act cac aac tac cag      626
   Asn Ala Trp Ala Lys Leu Gly Met Asn Leu Gly Thr His Asn Tyr Gln
   155                 160                 165                 170
   atc gtg gct acc gag ggt tac cag agc agt gga tct tcg tcc atc act      674
   Ile Val Ala Thr Glu Gly Tyr Gln Ser Ser Gly Ser Ser Ser Ile Thr
                   175                 180                 185
   gtt cag taagcggtgg atgtgtggat tgaacgattg tgcatgtaaa tactgagcag       730
   Val Gln
   tcgtatgata tgtgaaacag gtagttgttt ggtaccaatg tactggtcat ttggag        786
<210> 6
   <211> 225
   <212> PRT
   <213> Aspergillus niger DSM 12665
<400> 6
   Met Leu Thr Lys Asn Leu Leu Leu Cys Phe Ala Ala Ala Lys Ala Ala
           -35                 -30                 -25
   Leu Ala Val Pro His Asp Ser Val Ala Gln Arg Ser Asp Ala Leu His
       -20                 -15                 -10
   Met Leu Ser Glu Arg Ser Thr Pro Ser Ser Thr Gly Glu Asn Asn Gly
   -5                  -1  1               5                   10
   Phe Tyr Tyr Ser Phe Trp Thr Asp Gly Gly Gly Asp Val Thr Tyr Thr
               15                  20                  25
   Asn Gly Asp Ala Gly Ala Tyr Thr Val Glu Trp Ser Asn Val Gly Asn
           30                  35                  40
   Phe Val Gly Gly Lys Gly Trp Asn Pro Gly Ser Ala Gln Asp Ile Thr
       45                  50                  55
   Tyr Ser Gly Thr Phe Thr Pro Ser Gly Asn Gly Tyr Leu Ser Val Tyr
   60                  65                  70                  75
   Gly Trp Thr Thr Asp Pro Leu Ile Glu Tyr Tyr Ile Val Glu Ser Tyr
                   80                  85                  90
   Gly Asp Tyr Asn Pro Gly Ser Gly Gly Thr Tyr Lys Gly Thr Val Thr
               95                  100                 105
   Ser Asp Gly Ser Val Tyr Asp Ile Tyr Thr Ala Thr Arg Thr Asn Ala
           110                 115                 120
   Ala Ser Ile Gln Gly Thr Ala Thr Phe Thr Gln Tyr Trp Ser Val Arg
       125                 130                 135
   Gln Asn Lys Arg Val Gly Gly Thr Val Thr Thr Ser Asn His Phe Asn
   140                 145                 150                 155
   Ala Trp Ala Lys Leu Gly Met Asn Leu Gly Thr His Asn Tyr Gln Ile
                   160                 165                 170
   Val Ala Thr Glu Gly Tyr Gln Ser Ser Gly Ser Ser Ser Ile Thr Val
               175                 180                 185
   Gln
<210> 7
   <211> 1086
   <212> DNA
   <213> Aspergillus niger DSM 12665
<220>
   <221> CDS
   <222> (65)..(832)
<220>
   <221> sig_peptide
   <222> (65) .. (148)
<220>
   <221> mat_peptide
   <222> (149)..(832)
<400> 7
   caccctcaac tttcaagtca cagttgtacc cttctacttg gatatattct ttcttatcat      60
   catc atg gtg tct ttc ctt ggc caa gca cgc ctg gcc gtg cca atc ctg      109
        Met Val Ser Phe Leu Gly Gln Ala Arg Leu Ala Val Pro Ile Leu
                    -25                     -20                 -15
   tca gct ttt gcg tgc atg ctt gcg gca agt tcc gcc atc ccc cca cca      157
   Ser Ala Phe Ala Cys Met Leu Ala Ala Ser Ser Ala Ile Pro Pro Pro
               -10                 -5                  -1  1
   ccg cgt gga gct ctt tca ccc gaa cgt cta caa tgg att aga gag gtg      205
   Pro Arg Gly Ala Leu Ser Pro Glu Arg Leu Gln Trp Ile Arg Glu Val
       5                   10                  15
   atc ggc aat cag act gag aac gac agc gtt tcg gac ctt gct aag cgg      253
   Ile Gly Asn Gln Thr Glu Asn Asp Ser Val Ser Asp Leu Ala Lys Arg
   20                  25                  30                  35
   agc act atc ctg cat acc agc caa gac gga gtt gac agc gcc gga ttc      301
   Ser Thr Ile Leu His Thr Ser Gln Asp Gly Val Asp Ser Ala Gly Phe
                   40                  45                  50
   tac tac tca gta tac aat gac aac ggg gcc gat gtt gga tac acc gaa      349
   Tyr Tyr Ser Val Tyr Asn Asp Asn Gly Ala Asp Val Gly Tyr Thr Glu
               55                  60                  65
   tac ccc acg acc ggc cag ttc gaa ctt ggc tgg agt gct gag gcg gaa      397
   Tyr Pro Thr Thr Gly Gln Phe Glu Leu Gly Trp Ser Ala Glu Ala Glu
           70                  75                  80
   ttc ctc gct gga aag ggc ttc aag ggc ggc aac cca cgc tcc ttg acc      445
   Phe Leu Ala Gly Lys Gly Phe Lys Gly Gly Asn Pro Arg Ser Leu Thr
       85                  90                  95
   tgg gac ggt tat ttc acc gcc gaa ggg gac tgg act ttg gcc att tat      493
   Trp Asp Gly Tyr Phe Thr Ala Glu Gly Asp Trp Thr Leu Ala Ile Tyr
   100                 105                 110                 115
   ggc tgg acc aca aac ccc gtc acc gag tgg tat att gtg gag tcg cat      541
   Gly Trp Thr Thr Asn Pro Val Thr Glu Trp Tyr Ile Val Glu Ser His
                   120                 125                 130
   gga agt gga acc cca ggc aac ggg aac atc ctc ggc cag gtt gat agt      589
   Gly Ser Gly Thr Pro Gly Asn Gly Asn Ile Leu Gly Gln Val Asp Ser
               135                 140                 145
   gat ggc ggc gtc tac gat gtc tat aat ctc ccc tat aga aat gtt ccg      637
   Asp Gly Gly Val Tyr Asp Val Tyr Asn Leu Pro Tyr Arg Asn Val Pro
           150                 155                 160
   gag atc tac ggt gtc acc aac ttc gac cag cac tgg tcg gtg cgt cgc      685
   Glu Ile Tyr Gly Val Thr Asn Phe Asp Gln His Trp Ser Val Arg Arg
       165                 170                 175
   tcc cac cgc tct act ggc acc gtc gac gtg agc gcc cat ttc cag cgc      733
   Ser His Arg Ser Thr Gly Thr Val Asp Val Ser Ala His Phe Gln Arg
   180                 185                 190                 195
   tgg aag gaa ttg ggt ctc acc cct ggc tcc cca gtc ttc cag atg gtt      781
   Trp Lys Glu Leu Gly Leu Thr Pro Gly Ser Pro Val Phe Gln Met Val
                   200                 205                 210
   acc ctg gaa ggc ttt tcc ggc cag ggg tac ctg gac ttc acc gtt agt      829
   Thr Leu Glu Gly Phe Ser Gly Gln Gly Tyr Leu Asp Phe Thr Val Ser
               215                 220                 225
   gca tagaaaggtt cagggacctc gtggtctcag ctggcggcga gcacttactt           882
   Ala
   gaggccagat cccctccacg tgtatctttt tttctagggc aaagaatggt gacttcggtt    942
   caagaaacag aagcccccct ttcccttcct ttttgtttct tcttctctta gagcctgctc   1002
   tttaacaact tgttcgcttc ttttccagtt accaattatt ttttgacctt caaagatatt   1062
   tactatcgaa ctaatttgtt tatc                                          1086
<210> 8
   <211> 256
   <212> PRT
   <213> Aspergillus niger DSM 12665
<400> 8
   Met Val Ser Phe Leu Gly Gln Ala Arg Leu Ala Val Pro Ile Leu Ser
               -25                 -20                 -15
   Ala Phe Ala Cys Met Leu Ala Ala Ser Ser Ala Ile Pro Pro Pro Pro
           -10                 -5                  -1  1
   Arg Gly Ala Leu Ser Pro Glu Arg Leu Gln Trp Ile Arg Glu Val Ile
   5                   10                  15                  20
   Gly Asn Gln Thr Glu Asn Asp Ser Val Ser Asp Leu Ala Lys Arg Ser
                   25                  30                  35
   Thr Ile Leu His Thr Ser Gln Asp Gly Val Asp Ser Ala Gly Phe Tyr
               40                  45                  50
   Tyr Ser Val Tyr Asn Asp Asn Gly Ala Asp Val Gly Tyr Thr Glu Tyr
           55                  60                  65
   Pro Thr Thr Gly Gln Phe Glu Leu Gly Trp Ser Ala Glu Ala Glu Phe
       70                  75                  80
   Leu Ala Gly Lys Gly Phe Lys Gly Gly Asn Pro Arg Ser Leu Thr Trp
   85                  90                  95                  100
   Asp Gly Tyr Phe Thr Ala Glu Gly Asp Trp Thr Leu Ala Ile Tyr Gly
                   105                 110                 115
   Trp Thr Thr Asn Pro Val Thr Glu Trp Tyr Ile Val Glu Ser His Gly
               120                 125                 130
   Ser Gly Thr Pro Gly Asn Gly Asn Ile Leu Gly Gln Val Asp Ser Asp
           135                 140                 145
   Gly Gly Val Tyr Asp Val Tyr Asn Leu Pro Tyr Arg Asn Val Pro Glu
       150                 155                 160
   Ile Tyr Gly Val Thr Asn Phe Asp Gln His Trp Ser Val Arg Arg Ser
   165                 170                 175                 180
   His Arg Ser Thr Gly Thr Val Asp Val Ser Ala His Phe Gln Arg Trp
   185 190 195
   Lys Glu Leu Gly Leu Thr Pro Gly Ser Pro Val Phe Gln Met Val Thr
   200 205 210
   Leu Glu Gly Phe Ser Gly Gln Gly Tyr Leu Asp Phe Thr Val Ser Ala
   215 220 225
<210> 9
   <211> 214
   <212> PRT
   <213> unknown
<220>
   <223> Bacterium from environmental sample
<220>
   <221> SIGNAL
   <222> (1)..(29)
<220>
   <221> mat_peptide
   <222> (30) .. (214)
<400> 9
   Met Phe Lys Leu Ser Lys Lys Ile Leu Met Val Leu Leu Thr Ile Ser
   -25 -20 -15
   Met Ser Phe Ile Ser Leu Phe Thr Val Thr Ala Tyr Ala Ala Ser Thr
   -10 -5 -1 1
   Asp Tyr Trp Gln Asn Trp Thr Asp Gly Gly Gly Thr Val Asn Ala Thr
   5 10 15
   Asn Gly Ser Asp Gly Asn Tyr Ser Val Ser Trp Ser Asn Cys Gly Asn
   20 25 30 35
   Phe Val Val Gly Lys Gly Trp Thr Thr Gly Ser Ala Thr Arg Val Ile
   40 45 50
   Asn Tyr Asn Ala Gly Ala Phe Ser Pro Ser Gly Asn Gly Tyr Leu Ala
   55 60 65
   Leu Tyr Gly Trp Thr Arg Asn Ser Leu Ile Glu Tyr Tyr Val Val Asp
   70 75 80
   Ser Trp Gly Thr Tyr Arg Pro Thr Gly Thr Tyr Lys Gly Thr Val Thr
   85 90 95
   Ser Asp Gly Gly Thr Tyr Asp Ile Tyr Thr Thr Thr Arg Thr Asn Ala
   100 105 110 115
   Pro Ser Ile Asp Gly Asn Asn Thr Thr Phe Thr Gln Phe Trp Ser Val
   120 125 130
   Arg Gln Ser Lys Arg Pro Ile Gly Thr Asn Asn Thr Ile Thr Phe Ser
   135 140 145
   Asn His Val Asn Ala Trp Lys Ser Lys Gly Met Asn Leu Gly Ser Ser
   150 155 160
   Trp Ser Tyr Gln Val Leu Ala Thr Glu Gly Tyr Gln Ser Ser Gly Tyr
   165 170 175
   Ser Asn Val Thr Val Trp
   180 185